# EUROPEAN PATENT APPLICATION

(11) **EP 2 060 256 A1**
(43) Date of publication of application: **20.05.2009**
(21) Application number: 07022283.1
(22) Date of filing: 16.11.2007
(51) Int. Cl.: A61K 31/05, A61P 9/00, A61P 19/00

(54) **Pharmaceutical and nutraceutical compositions based on menaquinols**

(71) Applicant: Gnosis S.p.A., 20121 Milano (IT)
(72) Inventor: Valoti, Ermanno, 24044 Dalmine (Bergamo) (IT); Bianchi, Davide, 20100 Milano (IT)
(74) Representative: Minoja, Fabrizio

(57) **Abstract**

The invention relates to pharmaceutical and nutraceutical compositions containing as the active ingredient a menaquinol of formula I: 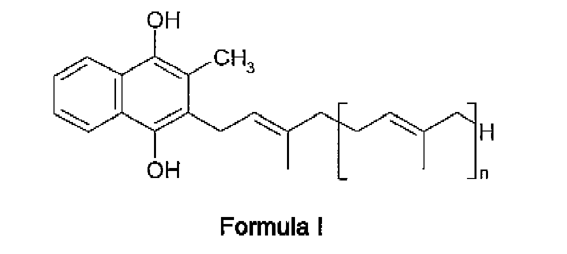 wherein n is as defined in the description,
for the prevention of cardiovascular diseases and bone metabolism disorders.

## Description

The present invention relates to compositions containing menaquinols as active ingredients.

The invention also relates to the use of menaquinols for the preparation of medicinal or nutraceutical products for the treatment and prevention of cardiovascular diseases and bone metabolism disorders.

### Background to the invention

Vitamin K2 or menaquinone, of the formula: 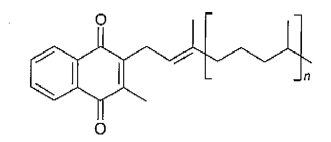 has long been studied due to its crucial role in controlling the physiological blood coagulation and bone metabolism processes. Dietary sources of vitamin K2 are eggs, dairy products, ox liver and fermented soya (natto).

The number of isoprenoid units ("n" in the formula shown above) can range between 2 and 12, although the terms from 4 to 7 are the most common, and generally referred to as MK-4, MK-5, MK-6 and MK-7. Menaquinones MK-4 and MK-7 (ie. those with 4 and 7 isoprenoid units, respectively) are the most abundant, and are available on the market.

Therapeutic uses of vitamin K have been described in numerous publications and patents, such as EP 679394, EP 1153548, EP 1728507, JP 11/127816, Int. J. Vitamin and Nutrition Res. 66 (1), 1996, 36-38, J. of Nutrition, 134(11), 2004, 3100-3105, and J. of Dairy Sci. 82(9), 1999, 1897-1903.

Reduced forms of menaquinones known as menaquinols were recently suggested for the topical cosmetic application (US 2007/0072940).

### Description of the invention

It has now been found that menaquinols, which can be represented by Formula I below: 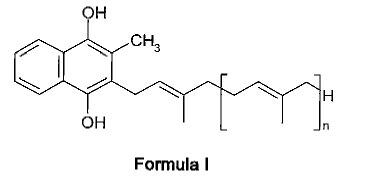 wherein n is an integer of 1 to 12, have more advantageous effects when administered orally, than the corresponding oxidised forms which have conventionally been used to date.

In particular, menaquinols posses improved pharmacokinetic properties and higher bioavailability than menaquinones.

The present invention, therefore, relates to dietary, pharmaceutical or nutraceutical compositions containing at least one menaquinol of formula I as the active ingredient, in admixture with suitable vehicles or excipients.

The invention also relates to the use of menaquinols of formula I for the preparation of medicinal or nutraceutical products for the treatment and prevention of cardiovascular diseases and bone metabolism disorders.

Menaquinols wherein n is an integer of 4 to 7 are preferred. Menaquinols deriving from the reduction of menaquinones MK-4 and MK-7 (Formula I, n=4 and n=7 respectively) are particularly preferred, and menaquinol MK-7 is even more preferable.

Menaquinols can be obtained by reduction with conventional methods starting from the natural commercially available menaquinones. Examples of reducing agents which can usefully be employed to prepare menaquinols from menaquinones include alkaline bisulphites, dithionites, metal hydrides and the like. The reaction can be carried out in solution or in suspension. Preferably, an aqueous solution of menaquinone in organic solvent is treated with an aqueous solution of reducing agent, such as sodium dithionite. After separation of the phases and evaporation of the organic solvent, the reduced menaquinols are isolated in crystalline form. The crystals obtained can then be mixed with fats, glycols or oils, in particular vegetable or animal oils such as soy oil, sunflower oil, olive oil, fish oil and the like. The oil can advantageously be added to the organic phase after reduction to provide, upon evaporation of the more volatile organic solvent (such as ethyl acetate, ethanol or methanol), a solution of menaquinols in oil which can be directly used to prepare the final formulations.

These solutions are preferred as they solve the problem of oxidation sensitivity of menaquinols.

The formulations according to the invention may take the form of gelatin capsules, tablets, liquid forms and the like. The oily solutions may possibly be added to foods or drinks or adsorbed on suitable solid media (cyclodextrins, modified starches or plant fibres).

The daily doses of menaquinols may range between 10 and 1000 µg, preferably 10 to 200 µg. In view of their high bioavailability, the doses will generally be lower than those recommended to date for menaquinones.

In addition to menaquinols, the compositions according to the invention may contain other ingredients with complementary or useful activities for the intended pharmaceutical, nutritional or nutraceutical uses. Examples of such ingredients are vitamin C, vitamins D, tocopherols, unsaturated fatty acids, trace elements, plant extracts, mineral salts, amino acids, carnosine, carnitine, oligosaccharides, etc..

The compositions according to the invention are useful for the treatment and prevention of cardiovascular diseases and bone metabolism disorders, with an efficacy similar to or greater than that known for vitamin K2 (menaquinone).

The following examples illustrate the invention in greater detail.

### Example 1

100 g of oxidised vitamin K₂-mK₄ and a solution of 100 g of sodium dithionite in 1000 ml of water were added to 1000 ml of ethyl acetate; the mixture was placed under stirring at room temperature until the reduction reaction was complete (two hours), and the phases were then separated. All operations were carried out under nitrogen atmosphere. The organic phase was concentrated under vacuum until complete removal of ethyl acetate, to obtain 100 g of reduced vitamin K₂-mK₄, as a white crystal.

The weight ratio of reduced to oxidised vitamin K₂-mK₄ in the crystals was 98.5:1.5.

### Example 2

100 g of oxidised vitamin K₂-mK₄ and a solution of 100 g of sodium dithionite in 1000 ml of water were added to 1000 ml of ethyl acetate; the mixture was placed under stirring at room temperature until the reduction reaction was complete (two hours), and the phases were then separated. 390 g of soya oil was added to the upper organic phase. All operations were carried out under nitrogen atmosphere. The suspension was concentrated under vacuum until complete removal of ethyl acetate, to obtain 400 g of a reduced solution of vitamin K₂-mK.

The weight ratio of reduced vitamin K₂-mK₄ to oxidised vitamin K₂-mK₄ in the crystals was 98.5:1.5.

### Example 3

10 g of the reduced vitamin K₂-mK₄ obtained in example 1 (the weight ratio of reduced vitamin K₂-mK₄ to oxidised vitamin K₂-mK₄ was 98.5:1.5) was added to 100 ml of ethanol at 25°C; the mixture was agitated at room temperature, and the reduced vitamin K₂-mK₄ dissolved completely. 40 ml of demineralised water was added, still under stirring. The solution was agitated and cooled to 0°C in 1 h. The temperature was maintained at 0°C for 1 h. The resulting mixture was then placed under vacuum, and the wet crystals were washed successively with cold methanol, cold water and cold methanol. The wet white crystals were resuspended in demineralised, degassed water and stored for the stability tests. All operations were carried out under nitrogen atmosphere. The starting weight ratio of reduced vitamin K_{z} to oxidised vitamin Kz in the crystals was 98.5:1.5.

### Example 4

10 g of the reduced vitamin K₂-mK₄ obtained in example 2 (the weight ratio of reduced vitamin K₂-mK₄ to oxidised vitamin K₂-mK₄ was 98.5:1.5) was added to 100 ml of ethyl acetate at 25°C; the mixture was agitated at 40°C, and the reduced vitamin K₂-mK₄ dissolved completely. 390 ml of soya oil was added, still under stirring. All the above operations were performed in a nitrogen atmosphere. The resulting suspension was concentrated under vacuum until complete removal of ethyl acetate, to obtain 400 g of an oxidised solution of vitamin K₂-mK₄.

The weight ratio of reduced vitamin K₂-mK₄ to oxidised vitamin K₂-mK₄ in the crystals was 81.0:19.0.

### Example 5

The reduced vitamin K₂ obtained in the preceding examples was kept in air for 4 days at 30°C, screened from light. The weight ratio of the reduced/oxidised vitamin K₂ was measured after 4 days. The results demonstrate that the reduced vitamin K_{z} solutions obtained by direct solubilisation in fat and oil are excellent in terms of stability compared with reduced vitamin K_{z} crystals which are crystallised from solvents, or solutions obtained from isolated crystals.

## Claims

1. Compositions containing as the active ingredient a menaquinol of formula I: 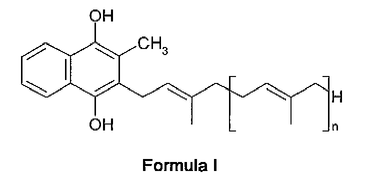 wherein n is an integer of 1 to 12, in admixture with suitable vehicles or excipients.

2. Compositions as claimed in claim 1 wherein, in the compound of formula I, n is an integer of 4 to 7.

3. Compositions as claimed in claim 2 wherein, in the compound of formula I, n is 4 or 7.

4. Compositions as claimed in any of claims 1 to 3, wherein the compound of formula I is in oily solution.

5. Compositions as claimed in any of claims 1 to 4, containing unit doses that provide 10 to 1000 µg, and preferably 10 to 200 µg a day of compound of formula I.

6. Use of a menaquinol of formula I for the preparation of medicinal or nutraceutical products for the treatment and prevention of cardiovascular diseases and bone metabolism disorders.
